# EUROPÄISCHE PATENTSCHRIFT

(11) **EP 2 368 637 B1**
(45) Veröffentlichungstag und Bekanntmachung des Hinweises auf die Patenterteilung: **20.11.2013**
(21) Anmeldenummer: 11158328.2
(22) Anmeldetag: 15.03.2011
(51) Int. Cl.: B01L 3/00, C12Q 1/00, G01N 33/50

(54) **Mikrofluidische Mehrfach-Messkammeranordnung**
Microfluidic multiple compartment measuring chamber assembly
Agencement de chambre de mesure multiple microfluidique

(30) Priorität: 16.03.2010 DE 102010002921
(43) Veröffentlichungstag der Anmeldung: 28.09.2011
(73) Patentinhaber: SensLab - Gesellschaft zur Entwicklung und Herstellung bioelektrochemischer Sensoren mbH, 04347 Leipzig (DE)
(72) Erfinder: Gründig, Bernd, 04318, Leipzig (DE); Wedig, Heiko, 04107, Leipzig (DE)
(74) Vertreter: Gulde Hengelhaupt Ziebig & Schneider

(56) Entgegenhaltungen:
- EP-A1- 1 413 879
- EP-A1- 1 912 074
- WO-A1-2006/074927
- US-A1- 2005 266 582

## Beschreibung

Die Erfindung betrifft eine mikrofluidische Mehrfach-Messkammeranordnung, die einen planaren Grundsensor und eine strukturierte Polymerfolie aufweist. Die dem Support zugewandte Unterseite der Folie besitzt unterschiedlich strukturierte Kammergeometrien, die vorzugsweise photolithographisch, im Mikrospritzguss-, im Tiefzieh- oder Heißprägeverfahren erzeugt werden. Besonders geeignet ist die erfindungsgemäße mikrofluidische Mehrfach-Messkammeranordnung für die Herstellung von Biosensoren, vorzugsweise in Form von Einmalgebrauchs- Enzym- und Affinitätssensoren.

### Stand der Technik

Mit der Nutzung diagnostischer Einmalgebrauchs-Sensoren auf der Grundlage enzymatischer und affinitätsbasierender Detektionsprinzipien, die eine unmittelbare, schnelle und quantitative Messung für Point of Care und Home Care-Anwendungen ermöglichen, sind Sensorstrukturen erforderlich, die einerseits eine möglichst richtige und reproduzierbare Messung sichern, andererseits aber kostengünstig herstellbar sein müssen. Diese Kriterien erfordern ein streng reproduzierbares Liquid-Handling der Probe, das gegebenenfalls Probeaufbereitungsschritte und -splitting sowie ein mehrkanaliges Ausmessen der Probe umfassen kann. Darüber hinaus sollte die erforderliche Probemenge so gering als möglich sein.

Sensorelemente, die in Kombination mit einem Handmessgerät eine einfache quantitative Vor-Ort-Messung ermöglichen, sind als so genannte "Einmal"- oder "Wegwerf"-Teststreifen oder -Sensoren bekannt. Insbesondere im Diabetesbereich haben sich derartige Messsysteme für Home Care - Anwendungen bewährt. In Eigenanwendung nutzen Diabetespatienten Glucoseteststreifen bzw. -sensoren zur Selbstkontrolle des Blutzuckerwertes.

In der Regel wird durch den Kontakt eines Kapillarbluttropfens an einer Probeaufnahmezone des Sensors ein Teil der Blutprobe durch Kapillarkraft auf eine interne Reagenzfläche des Sensors befördert. Auf der Reagenzfläche sind ein spezifisch die Glucose umsetzendes Enzym, ein Elektronenakzeptor und Additive zur Stabilisierung und schnellen Benetzbarkeit deponiert. Bei dem Enzym kann es sich um Oxidoreduktase wie Glucoseoxidase, Glucosedehydrogenase mit PQQ- oder FAD⁺ als prosthetischer Gruppe oder eine NAD'-abhängige Dehydrogenase handeln. Als Elektronenakzeptoren werden beispielsweise Chinone, chinoide Redoxfarbstoffe oder redoxaktive Metallkomplexe verwendet.

Die Oxidoreduktase reagiert mit der Blutglucose und überträgt ihre Elektronen, die bei dem Oxidationsprozess generiert werden, auf den Elektronenakzeptor, der damit equivalent zur Glucosekonzentration im Blut reduziert wird. Diese Redoxzustandsänderung des Elektronenakzeptors kann optisch oder elektrochemisch detektiert werden.

Bei einer elektrochemischen bzw. voltammetrischen Indikation des Elektronenakzeptors wird zwischen einer polarisierbaren Arbeitselektrode und einer Bezugselektrode eine Polarisationsspannung angelegt, die einen solchen Wert hat, dass an der anodisch geschalteten Arbeitselektrode der reduzierte Elektronenakzeptor zuverlässig oxidiert werden kann. Der resultierende Stromfluss im Außenkreis zwischen Arbeitselektrode und der Bezugselektrode ist aufgrund des stöchiometrischen Umsatzes proportional zur Zuckerkonzentration im Blut. Ein Handmessgerät stellt für die voltammetrische Messung des Sensors die erforderliche Polarisationsspannung bereit, misst den Signalstrom, ermittelt den Konzentrationswert, zeigt diesen auf dem LCD an und speichert den Messwert.

Für den Aufbau derartiger Einmalgebrauchs-Sensoren, die eine enzymatischvoltammetrische Indikation nutzen, ist eine Vielzahl technischer Lösungen bekannt.

Elektrochemisch-enzymatische Sensoren für den Einmalgebrauch bestehen prinzipiell aus einem planaren Grundsensor mit einer voltammetrischen Zwei- oder DreiElektrodenanordnung, die in einem "Messfenster" angeordnet ist, einschließlich Zuleitungen und elektrischen Kontaktflächen, einer das Messfenster bedeckenden Reagenzschicht, die das analyterkennende Enzym- oder Enzymsystem einschließlich Elektronenakzeptor sowie Additive enthält und einem Schichtaufbau um das Messfenster, der dem schnellen und definierten Zuführen der Probe dient.

Die Probe wird entweder direkt auf die Schichtenfolge gegeben oder mittels Kapillarkrafteffekt in einen Kapillarspalt, der über dem Messfenster angeordnet ist, auf die Reaktionsschicht gezogen.

Die zuletzt genannte technische Lösung wurde erstmals in EP 0 274 215 A1 beschrieben und wird derzeit am häufigsten für die Realisierung von Einmalgebrauchssensoren zur Blutzuckermessung angewandt.

Sowohl für zuverlässigere Messungen als auch für die parallele Bestimmung mehrerer Parameter mittels Einmalgebrauchssensoren sind neben konzentrationsabhängigen chemischen Größen physikalische Parameter wie beispielsweise Temperatur oder Leitfähigkeit zu bestimmen. In anderen Anwendungen ist es erforderlich, die Probe vorzubehandeln bzw. aufzubereiten oder bestimmten Wirkreagenzien auszusetzen. Dafür sind komplexe Strukturen zu realisieren, die außerdem sehr geringe Einzelkammervolumina erfordern sollten.

Es ist eine Vielzahl prinzipieller technischer Lösungen zur Generierung mikrofluidischer Anordnungen mit Multi-Messkammeranordnungen bekannt. Die Strukturierung der Messkammern, die mindestens einen Probeaufnahmeeingang und Ausgang bzw. eine Austrittsöffnung aufweisen kann beispielsweise durch anisotropes Ätzen bzw. Silizium-Tiefätzen (Advanced Silicon Etching) erfolgen. Die Abdeckung der Strukturen erfolgt beispielsweise mit Borsilikatglas durch anodisches Bonden, das ggf. auf seiner Innenseite mit einem entsprechenden Indikationssystem versehen wurde. Das Verfahren ist jedoch vergleichsweise kostenintensiv und erfordert Reinraumtechnologie. Für die kostengünstige Fertigung einfacher mikrofluidischer Disposables konnte sie sich bisher nicht durchsetzen.

Bei Verwendung von Kunststoff kann die Strukturierung auf der Grundlage von Mikrospritzguss, Heißprägen (DE 10 234 564 A1) oder durch neuere Lithographie- und laserstrukturierende Verfahren erfolgen (EP1 225 477A1, M.F. Jenen, J.E. McCormack, B. Helbo, L.H. Christensen, T.R.Christensen, N.J. Mikkelsen, and T. Tang. "Rapid Prototyping of Polymeric Microstructures with a UV Laser, Proc. CIRP seminar on micro and nano technology", Copenhagen, Denmark (2003)). Die mit den Kammergeometrien versehenen Strukturen, bei denen die Kammern parallel oder nacheinander angeordnet sind, werden dann über Stege oder Flächen, die die Messkammern umgeben, formschlüssig mit einem Grundsensor, auf dem die Indikationssysteme aufgebracht sind, durch Verkleben, Schweißen und Heißsiegeln verbunden.

Dabei besteht jedoch die Gefahr, dass aufgrund von Lösungsmitteln im Kleber oder des erforderlichen Wärmeeintrages beim Schweißen bzw. Heißsiegeln während des Fügeprozesses das bereits aufgetragene Indikationsreagenz auf dem Messfenster beschädigt wird. Es muss auf einen ausreichenden Abstand zwischen Fügeflächen und den mikrofludischen Strukturen geachtet werden, um ein Zulaufen der Kanäle durch Kleber, geschmolzenem Material und um ein wärmebedingtes Deformieren des den Mikrofluidkbereich begrenzenden Kunststoffs oder Spannungsbildungen zu verhindern. Die Verwendung eines Faserlasers mit dessen Hilfe Polycarbonat mit einer Schweißnahtbreite im Bereich von 100 µm verschweißt werden kann, ist ebenfalls beschrieben.

Bekannte technische Lösungen für Einmalgebrauchssensoren basieren immer noch auf Laminiertechniken die ausgestanzte Kunststofffolien und Kleberschichten miteinander kombinieren.

Die Patentschrift US 6 287 451 B2 beschreibt einen Sensoraufbau, der innerhalb eines kapillaren Messkammerkanals eine Kleberschicht mit drei nacheinander angeordneten kreisförmiger Öffnungen aufweist, in denen sich zwei Arbeitselektroden und eine Referenzelektrode befinden. Eine der beiden Arbeitselektroden ist mit dem analytdetektierenden Enzym beschichtet. Die drei Öffnungen mit den jeweils darin angeordneten Elektroden bilden separate Messzellhälften und sind über einen darüber verlaufenden Kanal, der einen Kapillarspalt bildet und sich bei der Messprozedur mit Probe befüllt, untereinander verbunden, so dass nacheinander enzymatisch- amperometrisch der Analyt und amperometrisch die elektrochemisch aktive Substanzen gemessen werden. Danach erfolgt über den Verbindungskanal zwischen einer der Arbeitselektroden und der Referenzelektrode eine Impedanzmessung zur Hämatokritbestimmung der Probe.

Die Strukturierbarkeit dieser Kammeranordnung ist auf die eingeschränkte Strukturierbarkeit der Klebefolien begrenzt. In diesem Zusammenhang können Alterungserscheinungen der Klebefolien wie Schrumpfung durch Lösungsmittelaustritt und die Änderung der Oberflächenbenetzbarkeit zu abweichendem Probebefüllungsverhalten und damit der Sensorcharakteristik führen. Probekammervolumina, die durch Strukturierung von Spacern und Klebefolien und deren laminierung realisiert werden, liegen derzeit bei minimal 0,3 µl.

Eine spezielle laminierte Mehrkammeranordnung für eine interne Kalibrierung und Funktionskontrolle auf dem Teststreifen beschreibt DE 60 2005 000 010 T2.

Es besteht aus einer optisch transparanten Grund- und Deckschicht, die sich über einen Spacer, der eine Diskontinuität aufweist, kongruent gegenüber stehen. Die Spacerschicht ist mit Grund- und Deckschicht über einen doppelseitigen Klebefilm verbunden, so dass eine Kavität gebildet wird. Innerhalb der Grund- und Deckfläche werden in der vorzugsweisen Ausführungsform auf den hydrophoben Oberflächen durch Auftragen einer hydrophilen, Schicht, die in Form von Stegen und punktförmigen Arealen strukturiert ist oder durch eine flächenstrukturierte Plasmabehandlung erhalten wird, ein System zur Probeverteilung- und Detektion geschaffen. Die Probe wird sich aufgrund der hohen Oberflächenenergie innerhalb der Kavität im Wesentlichen auf den hydrophilen, strukturierten Flächen, die seitlich jeweils von hydrophoben Bereichen mit niedriger Oberflächenenergie begrenzt werden bewegen. Auf den Detektionsarealen ist jeweils ein hydrophiles Reagenzgemisch deponiert. Von einer Probeaufnahmeöffnung aus wird die Probe kapillarkraftgetrieben über einen Probeverteilungspunkt zu den Detektionsarealen, die um den Knotenpunkt angeordnet sind, transportiert. Das Probevolumen im Bereich des Detektionsareals wird wesentlich von der Ausführung der hydrophilen bzw. hydrophoben Strukturierungen auf der Grund- und Deckfläche, deren Übergang und ihrer energetischen Eigenschaften bestimmt.

Gemäß der US2005/0208677 A1 wird zur Realisierung von Mehrfachmessungen auf Teststreifen eine poröse probetransportierende Schicht verwendet, über die mit einem vorgegebenen Abstand eine Folie mit Löchern oder mit quer zur Flussrichtung angebrachten Kanälen laminiert ist. Darüber ist eine weitere poröse Schicht auflaminiert, die jeweils unmittelbar über den Löchern bzw. Kanälen eine Reagenzschicht aufweist. Nach dem Auftragen der Probe an einem Ende der ersten porösen Schicht erfolgt ein lateraler Fluss, bei dem nacheinander die kapillaren Löcher oder Kanäle befüllt werden, über die die Probe die jeweilige Reagenzschicht erreicht. Auf diese Weise werden die Indikationsreaktionen sequentiell ausgelöst, wobei aufgrund der jeweiligen Kapillarräume keine Wechselwirkung zwischen den Reagenzbereichen eintreten kann. Der Aufbau besteht jedoch wieder aus einer Reihe verschiedener Schichten, die über Klebeverbindungen zu laminieren sind.

Die eingeschränkte Strukturierbarkeit der Klebefilme limitiert jedoch die Miniaturisierung der Fluidik, da einerseits komplexe Abläufe zur Probeaufbereitung und deren Ausmessung in Bezug auf mehrere Parameter erforderlich sind und andererseits nur eine begrenzte Probemenge zur Verfügung steht.

Dennoch erscheint aufgrund der Kompatibilität zu den etablierten Fertigungstechnologien ein Lösungsansatz unter weiterer Nutzung von Laminiertechnik als anstrebenswert, wenn es gelingt, die oben beschriebenen Nachteile zu überwinden, so dass auch mikrofluidische Strukturen aus Kunststofffolien für komplexere Applikationen in Form von EinmalgebrauchsSensoren massenproduktionstauglich nutzbar werden.

Aufgabe der Erfindung war es deshalb, einen mikrofluidischen Sensor, vorzugsweise zur Verwendung als Biosensor, für den Einmalgebrauch vorzugsweise unter Nutzung des Laminierens mittels doppelseitiger Klebefolien bereitzustellen, der die oben genannten Nachteile vermeidet und eine von Multikammeranordnungen mit Kammervolumen im Nanoliterbereich auf einem Sensor ermöglicht, die technologisch kostengünstig und streng reproduzierbar herstellbar sind. Darüber hinaus war abzusichern, dass Messprozeduren, die in benachbarten Kammern ablaufen, sich gegenseitig nicht beeinflussen.

Die Aufgabe der Erfindung wird gemäß des unabhängigen Anspruches 1 gelöst. Die Unteransprüche stellen bevorzugte Ausführungsvarianten dar.

Es wurde überraschenderweise gefunden, dass bei einer mikrofluidischen Mehrfach-Messkammeranordnung, die einen planaren Grundsensor 1 und eine strukturierte Polymerfolie 2 enthält und bei der die dem Grundsensor 1 zugewandte Polymerfolienoberfläche - die dem Grundsensor zugewandte Unterseite der Folie - gleich oder unterschiedlich vertieft strukturierte, parallel oder nacheinander angeordnete Kammergeometrien aufweisen, die Kammerwandungen 4a-d zwischen den jeweiligen Messkammern 3a-c auf der Grundsensoroberfläche ohne stoffschlüssigen Verbund aufsitzen, ohne dass bei der unmittelbar nach der Befüllung der Messkammern 3a-c mit Probe beginnenden Messsprozedur, die über 5 s bis 3 min erfolgen kann, eine merkliche Reagenzvermischung oder ein Austausch von Reaktionsprodukten zwischen den benachbarten Messkammern 3a-c auftritt, so dass sich die Einzelmessungen von benachbarten Messkammern 3a-c auf dem Grundsensor 1 gegenseitig nicht beeinflussen. Das heißt, die Unterseite der Polymerfolie 2 weist innerhalb des mikrofluidischen Flussweges mindestens zwei parallel angeordnete Vertiefungen auf, die über dem Sensor angeordnet sind und in Kombination mit diesem Grundsensor die Messkammern bilden. Die Messkammeranordnung hat bevorzugt einen Probenaufnahmeeingang und ggf. Ausgang. Die Vertiefungen befinden sich dazu z.B. an einem Ende der Folie, wo sie über dem Sensor so ausgerichtet sind, dass sich an der Kante eine Öffnung, z.B. in Form eines Spaltes, bildet, die den Probenaufnahmeeingang darstellen kann, durch welchen z.B. mittels Kapillarkräfte die zu bestimmende Proben in die so gebildeten Messkammern aufgenommen werden.

Die Kammerwandungen 4a-d zwischen diesen Vertiefungen, die mit dem Sensor die Messkammern 3a, b, c bilden, müssen eine Höhe aufweisen, die mindestens eine sichere Auflage auf der Oberfläche des Grundsensors 1 gewährleistet und maximal fünf Prozent über dieser Höhe liegen, die eine sichere Auflage gewährleistet. Die Dicke der Kammerwandungen 4a-d kann zwischen 25 µm und 250 µm und die Höhe der Kammerwandungen 4a-d zwischen 5 µm und 150 µm betragen. Die auf dem Grundsensor 1 aufliegenden Flächen der Kammerwandungen 4a-d können eben, halbkreisförmig oder spitz zulaufend ausgestaltet sein. Einzelne oder sämtliche vertieft strukturierten Kammergeometrien weisen Tiefen bevorzugt zwischen 5 µm und 150 µm auf.

Unter dem Begriff ,,Kammergeometrien" werden Kammern und Leitungen mit unterschiedlichen Formen (unabhängig von deren Tiefe) verstanden. Die dem Sensor zugewandte Seite der Polymerfolie weist (dreidimensionale) Vertiefungen in Form von Kammern bzw. Kammern und Leitungen mit unterschiedlichen Geometrien (= Formen) und Tiefen auf. Strukturierung bedeutet die entsprechenden Kammergeometrien mit unterschiedlichen Tiefen bereitzustellen. Diese Technik ist dem auf dem Gebiet der Mikrofluidik tätigen Fachmann bekannt.

Die strukturierte Polymerfolie 2 und der Grundsensor 1 sind außerhalb der vertieft strukturierten Kammergeometrien durch eine doppelseitige Klebefolie oder durch Verschweißen oder Versiegeln miteinander formschlüssig verbunden. Die Polymerfolie 2 und das Material des Grundsensors 1 können aus einem Polycarbonat, Polyester, Polystyren oder einem Polyacrylat bestehen. Die gleich oder unterschiedlich vertieft strukturierten, parallel oder nacheinander angeordneten Kammergeometrien werden vorzugsweise mittels Heißprägeverfahren, eines photolithographischen Verfahrens, Laserablation, Mikrospritzguss oder Tiefziehverfahren erzeugt. Bei dem Grundsensor 1 handelt es sich bevorzugt um einen elektrochemischen, mit leitfähigen, chemisch inerten Strukturen beschichteten Grundsensor oder um einen transparenten, für optische Messungen geeigneten Kunststoffsupport.

In einer einfachen Ausführungsform besteht der mikrofluidische Einmalgebrauchs-Sensor aus einer strukturierten Polymerfolie 2 und einem planaren amperometrischen Grundsensor 1, an dessen einem Ende parallel und symmetrisch zu dessen Längsachse eine rechteckförmige Doppelmesskammer wie folgt ausgebildet wird: Die dem Grundsensor 1 zugewandte Polymerfolienfläche weist an einem Ende, parallel und symmetrisch zu dessen Längsachse einen mittels Laserablation, Photolithographie oder Heißprägen erzeugten Steg auf, der die gemeinsame Kammerwandung 4a zwischen den beiden Messkammern 3a,b bildet. Die Kammerwandung 4a ist zwischen 25 µm und 250 *m stark und zwischen 5 µm und 150 µm hoch. Die zum Grundsensor 1 gerichtete Fläche der Kammerwandung, die auf dem Isolationslacksteg 8a des Grundsensors 1 zwischen den Messfenstern 7a,b des Grundsensors 1 aufliegt, ist eben gestaltet. Die Länge der Kammerwandung 4a entspricht der Länge der Messkammer und beträgt zwischen 1 mm und 5 mm.

Die Breite Länge und Höhe der zwei Messkammern wird durch eine rechteckförmige Aussparung 5a, die in eine doppelseitige Klebefolie 5 eingebracht wurde, festgelegt. Die Breite beträgt zwischen 0,5 mm und 5 mm und die Höhe zwischen 25 µm und 100 µm. Die rechteckförmige Aussparung 5a wird zur strukturierten Folie derart angeordnet, dass die Kammerwandung 4a nach dem Laminieren in der rechteckförmigen Aussparung 5a mittig positioniert ist. Die seitlichen äußeren Meßkammerwandungen werden durch die Ränder der doppelseitigen Klebefolie gebildet.

Der Grundsensor 1, weist zwei Messfenster 7a,b auf, die jeweils eine amperometrische Dreielektrodenanordnung aus einer Arbeits- Gegen- und Bezugselektrode (9a-c und 9d-f) enthalten. Die Messfenster 7a,b werden durch eine Isolationslackschicht 8, die im Bereich der Elektrodenanordnungen zwei symmetrisch angeordnete, rechteckförmige Aussparungen aufweist, gebildet. Die Aussparungen, die die Messfenster 7a,b darstellen, begrenzen die Elektrodenflächen 9a-f definiert. Zwischen den Messfenstern 7a,b verbleibt ein Steg aus Isolationslack 8a, der zwischen 50 µm und 500 µm breit ist.

Die Elektrodenflächen 9a-f sind mit Zuleitungen 10a-10f mit den Kontaktierungsflächen 11 a-f verbunden.

Elektrodenflächen 9a-f, Zuleitungen 10a-f und Kontaktflächen 11 a-f bestehen aus einer Kohlenstoffsiebdruckschicht oder einer inerten Edelmetalldünnschicht, die photolithographisch - galvanisch oder mittels Laser strukturiert sind. Sowohl die strukturierte Polymerfolie 2 als auch das Trägermaterial Material des Grundsensors 1 bestehen aus einem Polycarbonat, Polyester, Polypropylen, Polystyren oder einem Polyacrylat.

Auf die Messfenster 7a,b werden jeweils Reagenzschichten 6a,b aufgetragen, mit deren Hilfe eine Indikationsreaktion von Analyten oder Störkomponenten und anschließender Signalübertragung zur jeweiligen Elektrodenanordnung erfolgt.

Auf den Grundsensor 1 wird die mit doppelseitiger Klebefolie 5 versehene, strukturierte Polymerfolie 2 derart aufgebracht, dass die Kammerwandung 4a mittig zwischen den beiden Messfenstern 7a,b auf dem Isolationslacksteg 8a positioniert ist und die Klebefolienaussparung beide Messfenster einfasst. Die Dicke der doppelseitigen Klebefolie 5 entspricht maximal der Höhe der Kammerwandung 4a oder ist bis um fünf Prozent geringer als die Höhe der Kammerzwischenwandung. Auf diese Weise sitzt die Kammerzwischenwandung fest auf dem verbliebenen Isolationslacksteg 8a auf.

Wenn z.B. ein Blutstropfen an die spaltartige Öffnung der Sensorstirnseite des gelangt, wird aufgrund der Kapillarkraftwirkung soviel Probevolumen in die Messkammern3a,b gezogen, bis die Messkammern jeweils befüllt sind. Die durch das Blut verdrängte Luft der jeweiligen Messkammer entweicht über die Entlüftungskanäle 12a,b in eine Luftaustrittskammer 13 und die Blutprobe verbleibt ruhend in den Messkammern 3a,b. Während der unmittelbar darauf beginnenden Messprozedur, die bis zu drei Minuten andauern kann, wird keine Beeinflussung des jeweiligen Messignals aufgrund von Reagenzaustausch oder eines Reaktionsproduktaustausches zwischen den benachbarten Messkammern registriert.

Eine andere Ausführungsform des mikrofluidischen Einmalgebrauchs-Sensors, der in Bezug auf die verwendeten Materialien identisch mit denen der vorangegangenen Ausführungsform ist und aus einem planaren Grundsensor 1 und einer Polymerfolie 2 besteht, weist an einem Ende des Sensors drei parallel angeordnete Messkammern auf, die erfindungsgemäß durch Kammerwandungen 8b,c getrennt sind, die ohne stoffschlüssigen Verbund zum Grundsensor 1 auf diesem aufsitzen. Die Dreifachmesskammeranordnung ist symmetrisch zur Längsachse des Sensors angeordnet und schließt bündig mit der Stirnseite des Grundsensors, auf dem sich die Messfenster 7a-c befinden, ab.

Die dem Grundsensor 1 zugewandte rechteckförmige Polymerfolienfläche weist an einem Ende und parallel und symmetrisch zu dessen Längsachse drei rechteckige mittels Laserablation, Photolithographie oder Heißprägen erzeugte Vertiefungen zwischen 5µm und 100 µm auf, die die Messkammern 3a-c bilden und deren Wandungshöhe, mit Ausnahme der der Stirnseite, auf dem Niveau der Nullebene der Polymerfolie 2 entspricht. Die Breite der Kammerwandungen 8a,b beträgt zwischen 25 µm und 250 µm. An der Stirnseite, die zur Aufnahme der Probe vorgesehen ist, sind die Vertiefungen in den Messkammern 3a-c durchgehend und an der gegenüber liegenden Stirnseite sind die Wandungen jeweils mittig unterbrochen. Die Unterbrechungen gehen jeweils in sogenannte Entlüftungskanäle 14a-c über, die eine Breite von 10 µm bis 50 µm, eine Höhe von 25 µm bis 150 µm und einer Länge von 0,5 mm bis 2,0 mm aufweisen. Die Entlüftungskanäle 14a-c münden in einer großvolumigen Luftaustrittskammer 15 mit Verbindung zur Umgebungsatmosphäre.

An den jeweils äußeren Kammerwandungen der beiden außen angeordneten Messkammern schließt sich eine umlaufend und gegenüber der Foliennullebene vertiefte Fläche 16 an, deren Tiefe zwischen 20 µm und 100 µm beträgt. In diese vertiefte Fläche 16 wird eine doppelseitige Klebefolie 5 mit einer Dicke zwischen 20 µm und 100 µm eingelegt, die im Bereich der Messkammern eine Aussparung aufweist, so dass die von den äußeren Kammerwandungen 4a,d umgebenen Messkammern 3a-c in die Aussparung eingebettet werden.

Die äußeren begrenzenden Kammerwandungen 4a,d der Messkammern 3a und 3c als auch die inneren Kammerwandungen 4a,b zwischen den drei Messkammern 3a-c sind zwischen 25 µm und 500 µm stark und zwischen 5 µm und 150 µm hoch. Die auf dem Grundsensor 1 aufliegende Fläche der Kammerwände 4a-d ist spitz zulaufend. Die Länge der Kammerwandungen entspricht jeweils der Länge und Breite der Messkammern und beträgt zwischen 1 mm und 5 mm.

Die Länge, Breite und Höhe der drei Messkammern wird ausschließlich durch die Strukturierung der Polymerfolie 2 definiert.

Der rechteckförmige Grundsensor 1, weist zwei amperometrische Dreielektrodenanordnung mit jeweils einer Arbeits- Gegen- und Bezugselektrode (9a-f) und eine Elektrodenanordnung mit drei Elektroden zur Befüllungskontrolle und Leitfähigkeitsmessung (9a,g,h) sowie Zuleitungen 110a-h und Kontaktierungsflächen 11a-h auf. Eine nachfolgende Isolationslackschicht 8, die im Siebdruck aufgebracht ist, hat im Bereich der Elektrodenanordnungen drei parallel zueinander angeordnete rechteckförmige Aussparungen, die die Elektrodenflächen definiert begrenzen und jeweils ein Messfenster 7a-c bilden. Zwischen den Messfenstern 8a,b verbleibenden zwei Stege aus Isolationslack 8a,b, die zwischen 25 µm und 250 µm breit sind. Die Gesamtanordnung der Messfenster ist zwischen 1 mm und 2 mm breit sowie zwischen 2 mm und 5 mm lang. Die aus den drei rechteckförmigen Messfenstern 7a-c bestehende Anordnung schließt mit ihren schmalen Seiten an der Stirnseite des Sensors bündig ab und ist symmetrisch zur Längsseite des Sensors ausgerichtet.

Auf die Messfenster werden jeweils Reagenzschichten 6a-c aufgetragen, mit deren Hilfe eine Indikationsreaktion von Analyten und Störkomponenten und anschließender Signalübertragung zur jeweiligen Elektrodenanordnung erfolgt.

Auf den Grundsensor wird die mit doppelseitiger Klebefolie versehene, strukturierte Folie derart aufgebracht und laminiert, dass die drei Messkammern 3a-c jeweils über den drei Messfenstern 7a-c positioniert ist und die Klebefolienaussparung die Messfensteranordnung umfasst. Die Dicke der doppelseitigen Klebefolie 5 entspricht maximal der Tiefe der die äußeren Kammerwandungen umgebenden Fläche gegenüber der Foliennullebene und kann bis um fünf Prozent geringer als diese Tiefe sein. Auf diese Weise sitzen die Kammerzwischenwandung fest auf dem Isolationslack und den verbliebenen Isolationsschichtstegen 8a,b auf.

Der Probeaufnahme erfolgt über einen gemeinsamen Probeaufnahmespalt, der mit dem Ende des Grundsensors abschließt. Wenn z.B. ein Blutstropfen an die spaltartige Öffnung der Sensorstirnseite gelangt, wird aufgrund der Kapillarkraftwirkung soviel Probevolumen in die Messkammern 3a-c gezogen, bis die Messkammern jeweils befüllt sind. Die durch das Blut verdrängte Luft der jeweiligen Messkammer entweicht über die Entlüftungskanäle 12a-c in eine Luftaustrittskammer und die Blutprobe verbleibt ruhend in den Messkammern 3a-c. Während der Messprozedur, die bis zu drei Minuten andauern kann, wird keine Beeinflussung des jeweiligen Messignals aufgrund von Austausch von Reagenz oder Reaktionsprodukt zwischen den benachbarten Messkammern registriert.

Eine dritte Ausführungsform unterscheidet sich von der vorangegangenen dadurch, dass die strukturierte Klebefolie keine umlaufende Vertiefung um die Kammerwandungen zur Aufnahme einer Klebefolie aufweist, sondern die Fläche auf Nullebene verbleibt. Die formschlüssige Verbindung zwischen der strukturierten Polymerfolie 2 und dem Grundsensor 1 erfolgt auf der die äußeren Kammerwandungen umgebende Fläche 14 mittels Laserschweißen.

Die beschriebenen Ausführungsformen sind besonders für voltammetrische Einmalgebrauchs-Enzymsensoren, die parallel unterschiedliche Analyten oder die parallel Analyten und interferierende Komponenten bei Probevolumen zwischen 0,2 µl und 2 µl ausmessen und deshalb einen gut reproduzierbaren und fertigungstechnologisch einfachen und damit kostengünstigen Sensoraufbau erfordern. Durch die gleichzeitige Bestimmbarkeit des Analyten und der störenden Komponenten bei sehr geringem erforderlichen Probevolumen kann die Zuverlässigkeit der Messung von Einmalgebrauchs-Sensoren wesentlich verbessert werden, was im Bereich von Home Care-Diagnostik hilfreich ist aber auch für Anwendungen zur Bed-side - Messung beispielsweise von Blutzucker zunehmend Bedeutung im klinischen Alltag erlangt.

Aufgrund der erfindungsgemäßen Anordnung der Kammertrennwände, die ohne formschlüssige Verbindung auf dem Grundsensor aufsitzen, lassen sich in einfacher Weise Multikammeranordnungen in sehr kleinen Dimensionen realisieren, deren Kammerinhalte sich nach einer Befüllung bzw. während der sich anschließenden Messprozedur in den Kammern gegenseitig nicht beeinflussen.

Die erfindungsgemäße mikrofluidische Mehrfach-Messkammeranordnung lässt sich kostengünstig und massenproduktionsfähig strukturieren und ist geeignet für die einfache Realisierung von Mehrfach-Messkammeranordnungen auf planar strukturierten Sensoren zur parallelen Ausmessung z.B. mehrerer Substanzen oder Enzymaktivitäten in geringsten Probevolumina .

### Legende zu den Abbildungen

| Bezeichnungselement | Nr. |
|---|---|
| Grundsensor | 1 |
| Polymerfolie | 2 |
| Messkammer | 3a,b,c |
| Messkammerwandungen | 4a,b,c |
| doppelseitiger Klebefolie | 5 |
| Aussparung der doppelseitigen Klebefolie | 5a |
| Reagenzschicht | 6a-c |
| Messfenster | 7a,b,c |
| Isolationsschicht | 8 |
| Isolationsschichtsteg | 8a,b |
| Elektrodenflächen | 9a-h |
| Zuleitungen | 10a-h |
| elektrische Kontaktflächen | 11a-h |
| Entlüftungskanäle | 12a-c |
| Luftaustrittskammer | 13 |
| die äußeren Kammerwandungen umgebende Fläche | 14 |

Der erfindungsgemäße mikrofluidische Sensor wird durch nachfolgende Ausführungsbeispiele, und Zeichnungen näher erläutert.
Fig. 1 Querschnittsdarstellung einer mikrofluidischen Zweifach-Messkammeranordnung mit Grundsensor 1, strukturierter Polymerfolie 2, Messkammern 3a,b Messkammerwandung 4a mit ebener Auflagefläche, doppelseitiger Klebefolie 5 und Reagenzschicht en6a,b
Fig. 2 Querschnittsdarstellung einer mikrofluidischen Dreifach-Messkammeranordnung mit Grundsensor 1, strukturierter Polymerfolie 2, Messkammern 3a-c, Messkammerwandungen mit spitz zulaufenden Auflageflächen 4a-d, doppelseitiger Klebefolie 5 und Reagenzschichten 6a-c
Fig. 3 Querschnittsdarstellung einer mikrofluidischen Dreifach-Messkammeranordnung mit Grundsensor 1, strukturierter Polymerfolie 2, Messkammern 3a,b, Messkammerwandung mit abgerundeten Auflagefläche 4a, Schweißfläche 14 und Reagenzschicht 6a,b.
Fig. 4 Explosionsdarstellung eines Einmalgebrauchs-Enzymsensors mit einer mikrofluidischen Zweifach-Messkammeranordnung bestehend aus einem Grundsensor 1 mit Arbeits- Gegen- und Referenzelektrodenflächen 9a-f, Zuleitungen, 10a-f, Kontaktflächen 11 a-f, Isolationslack 8 und Isolationslacksteg 8a, Messfenstern 7a,b, Reagenzschichten 6a,b, doppelseitiger Klebefolie 5 mit Aussparung 5a, und strukturierter Polymerfolie 2 mit Messkammern 3a,b, Messkammerwandung 8a, Entlüftungskanälen 12a,b und Luftaustrittskammer 13
Fig. 5 Explosionsdarstellung eines Einmalgebrauchs-Enzymsensors mit einer mikrofluidischen Dreifach-Messkammeranordnung bestehend aus einem Grundsensor 1 mit Arbeits- Gegen- und Referenzelektrodenflächen 9a-h, Zuleitungen 10a-h, Kontaktflächen 11 a-h, Isolationslack 8 und Isolationslackstegen 8a,b, Messfenstern 7a-c, Reagenzschichten 6a-c, doppelseitiger Klebefolie 5 mit Aussparung 5a, und strukturierter Polymerfolie 2 mit Messkammern 3a-c, Messkammerwandungungen 4a-d, Entlüftungskanälen 12a-c und Luftaustrittskammer 13.

### Beispiel 1

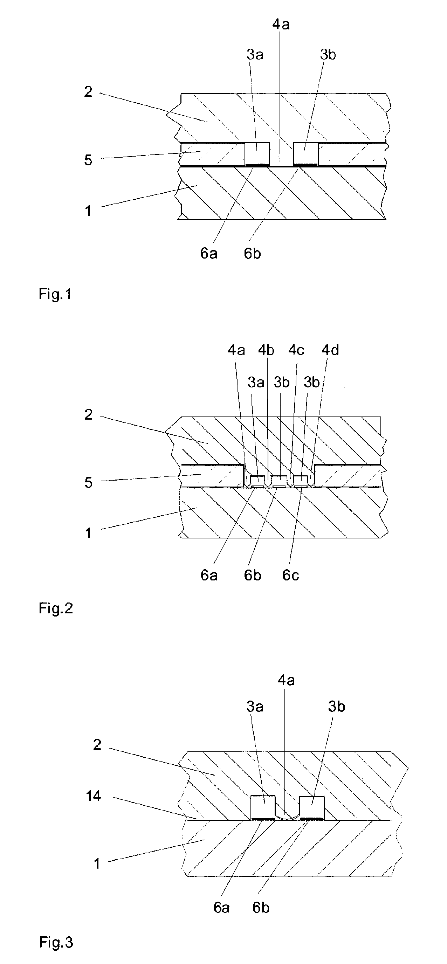

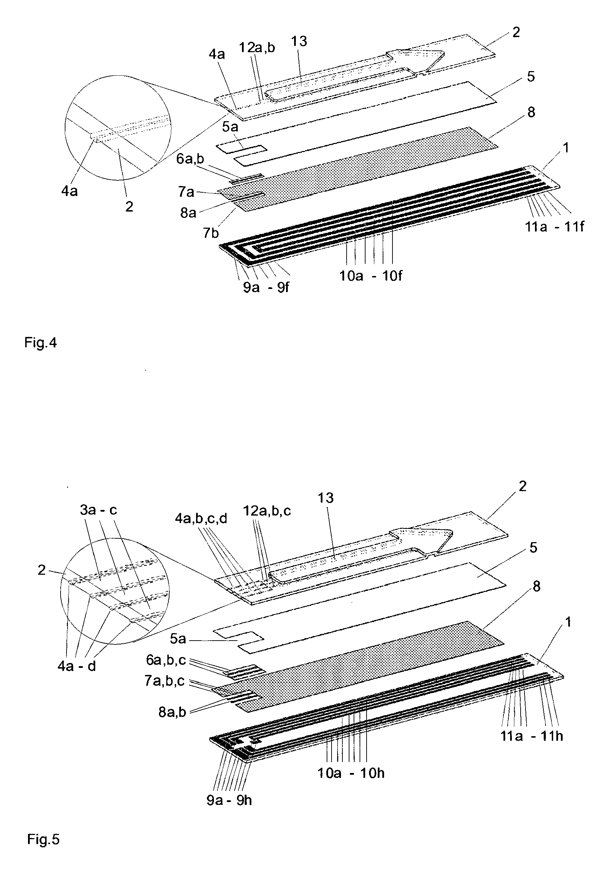

Erfindungsgemäßer mikrofluidischer Einmalgebrauchssensor zum parallelen Nachweis von Glucose und Laktat. Zur Erläuterung dienen Fig. 1 und Fig. 4.

Auf einem Polyester (Melinex) - Kunststoffsupport mit einer Dicke von 0,25 mm werden in Zehnernutzen mittels Dickschichttechnik ein Kohlenstoffdruck zur Erzeugung von zwei amperometrischen Drei-Elektroden-Anordnungen mit entsprechenden Elektrodenflächen 9a-f, Zuleitungen 10a-f und Kontaktierungsflächen 11 a-f unter Verwendung von Acheson PE 401-Kohlenstoffpaste (Acheson NL) und nach Zwischentrocknung ein Isolationslack 8 (240 SB, ESL Europe), wie in Fig. 4 dargestellt, aufgedruckt und bei 70°C gehärtet.

Der Isolationslack weist im Bereich der Elektrodenanordnung zwei parallel zueinander angeordnete Aussparungen von jeweils 0,5 mm x 3,5 mm (BxL) auf, so dass diese Aussparung, die die Messfenster 7a und 7b darstellen, die Elektrodenflächen in ihrer Breite definiert begrenzen. Dementsprechend betragen die Einzelflächen von Arbeits-, Referenz-und Gegenelektrode 9a-f, die in beiden Messfenstern 7a,b nacheinander angeordnet sind, jeweils 0,5 mm²_{.}

Der verbleibende Steg aus Isolationslack 8a zwischen den Messfenstern beträgt 0,25 mm.

Mittels eines Dispensers werden 0,3 µl einer Reaktionslösung bestehend aus 2 Units Glucoseoxidase (Roche), 140 µg Ferricyanid (Sigma), 1,6 µg Triton X 100 (Sigma) und 1,5 µg mikrokristalliner Zellulose (Aldrich) als Reaktionsschicht 6a zur Indikation von Glucose in 0,02 µl Dispensierschritten jeweils über das gesamte Messfenster 7a der Zehnernutzen gleichmäßig verteilt aufgetragen.

Nach Wechsel des Dispensereinsatzes werden 0,3 µl einer Reaktionslösung bestehend aus 1,5 Units Lactatoxidase (Roche), 140 µg Ferricyanid (Sigma), 1,6 µg Triton X 100 (Sigma) und 1,5 µg mikrokristalliner Zellulose (Aldrich) als Reaktionsschicht 6b zur Indikation von Lactat in 0,02 µl Dispensierschritten jeweils über das gesamte Messfenster 7b der Zehnernutzen gleichmäßig verteilt aufgetragen. Beide Reagenzlösungen werden über 50 min bei 35 °C getrocknet.

Als Polymerfolie wird eine Polycarbonat - Folie von 0,25 mm Dicke verwendet, die mittels einer Stahlpatrize in einem Heißprägeprozess strukturiert wird. Die als Zehnernutzen hergestellte Patrize weist jeweils pro Nutzen eine Vertiefung in Form der Kammerwandung 4a und Erhebungen zur Ausbildung der beiden Entlüftungskanäle 12a,b und gemeinsamen Luftaustrittskammer 13 auf.

Eine doppelseitige Klebefolie 5 mit einer Dicke von 50 µm wird im Bereich der Messfenster mit einem Stanzwerkzeug, das 10 Stanzeinsätze aufweist, in den Abmaßen von 1,25 mm x 3,5 mm ausgespart. Bei entsprechend kontrolliertem Auflegen und Laminieren von Klebefolie 5 und strukturierter Polymerfolie 2 fasst die Aussparung 5a die beiden Messkammern 3a,b ein und die Kammerwandung 4a der strukturierten Polymerfolie wird jeweils symmetrisch innerhalb der Ausstanzung der Klebefolie 5a und auf dem Isolationslacksteg 8a positioniert, so dass nach dem Laminieren in Kombination von Grundsensor 1, Aussparung der doppelseitigen Klebefolie 5a und der in der strukturierten Polymerfolie 2 geprägten Kammerwandung 8a zwei Messkammern mit den Dimensionen von jeweils 0,5 mm x 3,5 mm x 0,05 mm (BxLxH) entstehen. Nach einer Zeit von 24 h hat die Klebefolie die geprägte Folie und den Grundsensor soweit angezogen, dass die Kammerwandung zuverlässig auf dem Isolationslacksteg 8a aufsitzt. Danach wird der Nutzen durch Schneiden in zehn Sensoren mit Abmaßen von 6 mm x 36 mm (B x L) vereinzelt.

Zur Durchführung von Kontrollmessungen werden die Sensoren über die Kontaktierungsflächen jeweils an eine potentiostatische Ausleseeinheit mit zwei Messkanälen (Handmessgerät SensLab) unter Verwendung einer Polarisationsspannung von +450 mV angeschlossen und nacheinander mit einer 0,1 M Phosphatpufferlösung pH 6,8, einer 0,1 M Phosphatpuffer - Kontrolllösung mit 10 mM Glucose, pH 6,8, einer 0,1 M Phosphatpuffer - Kontrolllösung mit 10 mM Laktat, pH 6,8 und schließlich mit frischem Kapillarblut ausgemessen.

**Tabelle 1: Ergebnisse der Kontrollmessungen mit Doppelkammersensoren; jeder Wert stellt den Mittelwert aus einer Doppelbestimmung dar**

| Probe | Glcuosesensor (Messkammer 7a) | Laktatsensor (Messkammer 7b) |
|---|---|---|
| Pufferlösung | 1,3 µC | 1,7 µC |
| | | |
| 10 mM Glucoselösung | 23,2 µC | 1,5 µC |
| | | |
| 10 mM Laktatlösung | 1,4 µC | 33,4 µC |
| | | |
| Frisches Kapillarblut | 12,7 µC | 5,6 µC |

Die parallel registrierten Messströme wurden über eine Zeitdauer von 15 s gemessen und integriert, so dass die Messwerte als Ladungen angezeigt wurden. Die Ergebnisse, die in Tabelle 1 dargestellt sind, weisen darauf hin, dass keine wechselseitige Beeinflussung der Messungen aufgrund von Reagenz- oder Reaktionsproduktaustausch zwischen den benachbarten Messkammern vorlag.

### Beispiel 2

Erfindungsgemäßer mikrofluidischer Einmalgebrauchssensor zur Bestimmung von Glucose, Laktat und Hämatokrit. Zur Erläuterung dienen Fig. 2, und Fig. 5.

Auf einem Polyester (Melinex) - Kunststoffsupport mit einer Dicke von 0,25 mm, das mit einer im PVD-Verfahren aufgebrachten Goldschicht von ca. 50 nm versehen ist, werden als Zehnersensornutzen mittels Flächen-Laserablation jeweils pro Sensor zwei amperometrische Drei-Elektroden-Anordnungen mit Elektrodenflächen 9a-f sowie eine Zwei-Elektroden-Leitfähigkeitsanordnung mit Elektrodenflächen 9a,g,h einschließlich entsprechende Zuleitungen 10a-h und Kontaktierungsflächen 11 a-h erzeugt. Danach wird ein Isolationslack 8 (240 SB, ESL Europe), wie in Fig. 5 dargestellt, aufgedruckt und bei 70°C gehärtet. Der Isolationslack 8 weist im Bereich der Elektrodenanordnung drei parallel zueinander angeordnete Aussparungen von jeweils 0,2 mm x 3,5 mm (BxL) auf, so dass diese Aussparungen, die die Messfenster 7a -c darstellen, die Elektrodenflächen in ihrer Breite definiert begrenzen. Die Breite der verbleibenden Stege aus Isolationslack 8a,b zwischen den Messfenstern beträgt 0,15 mm.

Die Einzelflächen von Arbeits-, Referenz- und Gegenelektrode 11 a-f der amperometrischen Dreielektrodenanordnungen, die in beiden Messfenstern 7a,c nacheinander angeordnet sind, betragen jeweils 0,2 mm². Die Elektrodenflächen im Messfenster 7b zur Leitfähigkeitsmesssung betragen 0,15 mm².

Mittels eines Dispensers werden 0,1 µl einer Reaktionslösung bestehend aus 2 Units Glucoseoxidase (Roche), 140 µg Ferricyanid (Sigma), 1,6 µg Triton X 100 (Sigma-Aldrich) und 1,5 µg mikrokristalliner Zellulose (Aldrich) als Reaktionsschicht 6a zur Indikation von Glucose in 0,01 µl Dispensierschritten jeweils über das gesamte Messfenster 7a der Zehnernutzen gleichmäßig verteilt aufgetragen.

Nach Wechsel des Dispensiereinsatzes werden 0,1 µl einer Reaktionslösung bestehend aus 1,5 Units Lactatoxidase (Roche), 140 µg Ferricyanid (Sigma), 1,6 µg Triton X 100 (Sigma-Aldrich) und 1,5 µg mikrokristalliner Zellulose (Aldrich) als Reaktionsschicht 6c zur Indikation von Lactat in 0,01 µl Dispensierschritten jeweils über das gesamte Messfenster 7c der Zehnernutzen gleichmäßig verteilt aufgetragen.

Nach einem weiteren Wechsel des Dispensiereinsatzes werden schließlich 0,05 µl einer 1 % igen Detergenzlösung (Triton X 100, Sigma-Aldrich) als Reaktionsschicht 6b für eine Leitfähigkeitsmessung der Probe in 0,01 µl Dispensierschritten jeweils über das gesamte Messfenster 7c der Zehnernutzen gleichmäßig verteilt aufgetragen.

Die Reagenzlösungen werden über eine Zeitdauer von 50 min bei 35 °C getrocknet.

Als Polymerfolie wird eine Polycarbonat - Folie von 0,25 mm Dicke verwendet, die mittels einer Stahlpatrize in einem Heißprägeprozess strukturiert wird. Die als Zehnernutzen hergestellte Patrize weist Erhebungen für drei Messkammern 3a-c in den Maßen von jeweils 0,2 mm x 3,5 mm x 0,025 mm (BxLxH), Entlüftungskanäle 12a-c und eine Luftaustrittskammer 14 auf sowie vier auf Nullebene verbliebene Kammerwandungen 4a-d mit einer Dicke von jeweils 0,15 mm. Die Kammerwandungen 4a-d erhalten durch ein geringes umlaufendes Anphasen der Erhebungen für die Messkammern auf der Patrizze nach dem Abprägen spitz zulaufende Flächen. Den äußeren Kammerwandungen 4a,d schließt sich eine flächige Erhebung von 50 µm an. Die Erhebung auf der Patrize führt in der Abprägung der Polymerfolie zu einer gegenüber der Nullebene der Folie vertieften Fläche, die mit Ausnahme der Messkammern 3a-d, Kammerwandungen 4a-d, Entlüftungskanäle 14a-c und Luftaustrittskammer 15 die gesamte Folienfläche betrifft. Die Dimensionen der Entlüftungskanäle 12a-c betragen 50 µm x 100 µm x 1 mm (H x B x L) und die der Luftaustrittskammer 13 15 0,250 mm x 3 mm x 25 mm (H x B x L).

Eine doppelseitige Klebefolie 5 mit einer Dicke von 50 µm wird im Bereich der Messfenster mit einem Stanzwerkzeug, das 10 Stanzeinsätze aufweist und in den Abmaßen von 1,3 mm x 3,5 mm ausgespart. Nach dem Auflegen der doppelseitigen Klebefolie auf die vertiefte Fläche der strukturierten Folie, die dann mit den spitz zulaufenden Enden der Kammerwandungen 4a-d eine Ebene bildet, fasst deren Aussparung 5a bei entsprechend kontrolliertem Auflegen der Klebefolie 5 die drei Messkammern 3a-c einschließlich der Kammerwandungen 4a-d ein. Schließlich erfolgt das definierte Aufsetzen und Laminieren der Folien auf den Grundsensor derart, dass die in die Polymerfolie eingeprägten Messkammern 3a-c über den jeweiligen Messfenstern 7a-c des Grundsensors 1 und die inneren Kammerwandungen 4b,c über den Isolationslackstegen 8a,b positioniert werden.

Nach einer Zeit von 24 h hat die Klebefolie die geprägte Folie und den Grundsensor soweit angezogen, dass die Kammerwandungen zuverlässig auf den Isolationslackstegen 8a,b aufsitzen.

Danach wird der Nutzen durch Schneiden in zehn Sensoren mit Abmaßen von 6 mm x 36 mm (B x L) vereinzelt.

Zur Durchführung von Kontrollmessungen werden die Sensoren über die Kontaktierungsflächen an eine potentiostatische Ausleseeinheit mit zwei potentiostatischen Messkanälen sowie einen Leitfähigkeitsmesskanal (Handmessgerät SensLab) angeschlossen. Die Polarisationsspannung für die amperometrischen Messungen beträgt +450 mV.

Es wurden mit den Sensoren nacheinander entionisiertes Wasser, eine 0,01 M Kaliumchloridlösung, Kontrolllösungen mit 10 mM Glucose in 0,01 M Kaliumchloridlösung, 10 mM Laktat in 0,01 M Kaliumchloridlösung und schließlich frisches Kapillarblut ausgemessen.

**Tabelle 2: Ergebnisse der Kontrollmessungen mit von Sensoren mit Dreifachmesskammern; jeder Wert stellt den Mittelwert aus einer Doppelbestimmung bei 25 °C dar**

| **Probe** | **Glcuosesensor (Messkammer 7a)** | **Laktatsensor (Messkammer 7c)** | **Leitfähigkeit (Messkammer 7b)** |
|---|---|---|---|
| entionisiertes Wasser | 0,3 µC | 0,4 µC | 20 µS/cm |
| | | | |
| 0,01 M KCI | 0,8 µC | 0,7 µC | 1,5 mS/cm |
| | | | |
| 10 mM Glucose in 0,01 M KCI-Lösung | 12,2 µC | 0,8 µC | 1,7 mS/cm |
| | | | |
| 10 mM Laktat in 0,01 M KCI-Lösung | 0,3 µC | 21,7 µC | 1,6 mS/cm |
| | | | |
| Frisches Kapillarblut | 6,6 µC | 3,1 µC | 46 mS/cm |

Die parallel registrierten Messströme der amperometrischen Messungen wurden über eine Zeitdauer von 15 s gemessen und integriert, so dass die Messwerte als Ladungen angezeigt wurden. Die Leitfähigkeitsmessung erfolgte 5 s nach Beginn der Messprozedur über 2 s. Die Ergebnisse (Tabelle 2) weisen darauf hin, dass keine merkliche wechselseitige Beeinflussung der Messungen aufgrund des Austauschs von Reagenzien oder Reaktionsprodukt zwischen den benachbarten Messkammern vorlag.

### Beispiel 3

Erfindungsgemäßer mikrofluidischer Einmalgebrauchssensor zur Bestimmung von Glucose und interferierender Substanzen (Ascorbat, Harnsäure). Zur Erläuterung dienen Fig. 3, Fig. 4 und Fig.5.

Eine dritte Ausführungsform unterscheidet sich von der vorangegangenen dadurch, dass die strukturierte Klebefolie keine umlaufende Vertiefung um die Kammerwandungen zur Aufnahme einer Klebefolie aufweist, sondern die Fläche auf Nullebene verbleibt. Die formschlüssige Verbindung zwischen der strukturierten Polymerfolie 2 und dem Grundsensor 1 erfolgt entlang der äußeren Kammerwandungen mittels Laserweißen.

Auf einem Polyester (Melinex) - Kunststoffsupport mit einer Dicke von 0,25 mm werden in Zehnernutzen auf der Grundlage der Dickschichttechnologie ein Kohlenstoffdruck zur Erzeugung von zwei amperometrischen Drei-Elektroden-Anordnungen mit entsprechenden Elektrodenflächen 9a-f, Zuleitungen 10a-f und Kontaktierungsflächen 13a-f unter Verwendung von Acheson PE 401-Kohlenstoffpaste (Acheson NL) und nach Zwischentrocknung ein Isolationslack 8 (240 SB, ESL Europe), wie in Fig. 4 dargestellt, aufgedruckt und bei 70°C gehärtet.

Die Einzelflächen von Arbeits-, Referenz- und Gegenelektrode 9a-f, die in beiden Messfenstern 7a,b nacheinander angeordnet sind, betragen jeweils 0,5 mm². Der Isolationslack 8 weist im Bereich der Elektrodenanordnung zwei parallel zueinander angeordnete Aussparungen von jeweils 0,5 mm x 3,5 mm (BxL) auf, so dass diese Aussparungen, die die Messfenster 7a und 7b darstellen die Elektrodenflächen in ihrer Breite definiert begrenzen. Der verbleibende Steg aus Isolationslack 8a zwischen den Messfenstern beträgt 0,25 mm.

Mittels eines Dispensers werden 0,3 µl einer Reaktionslösung bestehend aus 2 Units Glucoseoxidase (Roche), 140 µg Ferricyanid (Sigma), 1,6 µg Triton X 100 (Sigma) und 1,5 µg mikrokristalliner Zellulose (Aldrich) als Reaktionsschicht 6a zur Indikation von Glucose in 0,02 µl Dispensierschritten jeweils über das gesamte Messfenster 7a der Zehnernutzen gleichmäßig verteilt aufgetragen.

Nach Wechsel des Dispensereinsatzes werden 0,3 µl einer Reaktionslösung bestehend aus 140 µg Ferricyanid (Sigma), 1,6 µg Triton X 100 (Sigma) und 1,5 µg mikrokristalliner Zellulose (Aldrich) als Reaktionsschicht 6b zur Indikation elektrochemisch aktiver Substanzen in 0,02 µl Dispensierschritten jeweils über das gesamte Messfenster 7b der Zehnernutzen gleichmäßig verteilt aufgetragen. Beide Reagenzlösungen werden über 50 min bei 35 °C getrocknet.

Als Polymerfolie wird eine Polycarbonat - Folie von 0,25 mm Dicke verwendet, die mittels einer Stahlpatrize in einem Heißprägeprozess strukturiert wird. Die als Zehnernutzen hergestellte Patrize weist jeweils pro Nutzen eine Vertiefung in Form der Kammerwandung 4a und Erhebungen zur Ausbildung zweier rechteckförmiger Messkammern 3a,b sowie von jeweils dazugehörigem Entlüftungskanal 12a,b und einer gemeinsamen Luftaustrittskammer 13 auf. Die parallel angeordneten rechteckförmigen Erhebungen auf der Patrize für die Messkammern 3a,b sind im Abstand von 0,2 mm angeordnet und weisen am längsseitigen Fuß jeweils eine abgerundete Phase auf, so dass nach dem Abprägen der Folie ein Steg bzw. eine Kammerwandung 4a zwischen den Messkammern mit einer Breite von 0,2 mm und abgerundeter Oberfläche entsteht. Die Kammergeometrie beträgt jeweils 0,5 mm x 3,5 mm x 0,05 mm (BxLxH).

Die strukturierte Polymerfolie 2 wird derart über dem Grundsensor 1 positioniert, dass die beiden geprägten Messkammern 3a,b passgenau über den Messfenstern und die Kammerwandung 4a auf dem Isolationslacksteg 8a positioniert werden.

Im Anschluss wird mittels Bündel-Laser in einer Fixiervorrichtung die Polymerfolie 2 entlang der Ränder der Messkammern 3a,b verschweißt. Durch die Oberflächenverschmelzung der Polymerfolie in der unmittelbaren Umgebung der Messkammern 14 erfolgt eine Absenkung der Messkammerhöhe um 2 bis 5 µm, die dazu führt, dass die inne liegende Kammerwandungung 4a zwischen den Messkammern 3a,b fest auf dem darunter befindlichen Isolationslacksteg 8a aufsitzt.

Danach wird der Nutzen durch Schneiden in zehn Sensoren mit Abmaßen von 6 mm x 36 mm (B x L) vereinzelt.

Zur Durchführung von Kontrollmessungen werden die Sensoren über die Kontaktierungsflächen an eine potentiostatische Ausleseeinheit mit zwei Messkanälen (Handmessgerät SensLab) unter Verwendung einer Polarisationsspannung von +450 mV angeschlossen. Es werden die Sensoren nacheinander mit einer 0,1 M Phosphatpufferlösung pH 6,8, einer Kontrolllösung mit 10 mM Glucose in 0,1 M Phosphatpuffer pH 6,8, einer 0,1 M Phosphatpufferlösung pH 6,8 mit 0,5 mM Ascorbat und schließlich mit Pufferlösung, die 10 mM Glucose und 0,5 mM Ascorbat enthielt, ausgemessen.

**Tabelle 3: Ergebnisse der Kontrollmessungen mit Doppelkammersensoren; jeder Wert stellt den Mittelwert aus einer Doppelbestimmung dar**

| Probe | Glcuosesensor (Messkammer 7a) | Interferenzsensor (Messkammer 7b) |
|---|---|---|
| Pufferlösung | 1,4 µC | 1,2 µC |
| | | |
| 10 mM Glucoselösung | 18,6 µC | 1,1 µC |
| | | |
| 0,5 mM Ascorbatlösung | 7,3 µC | 8,4 µC |
| | | |
| Pufferlösung mit 10 mM Glucose und 0,5 mM Ascorbat | 24,1µC | 8,1 µC |

Die parallel registrierten Messströme wurden über eine Zeitdauer von 15 s gemessen und integriert, so dass die Messwerte als Ladungen angezeigt wurden. Die Ergebnisse, die in Tabelle 3 dargestellt sind, weisen darauf hin, dass keine wechselseitige Beeinflussung der Messungen aufgrund von Reagenz- oder Reaktionsproduktaustausch zwischen den benachbarten Messkammern 7a,b vorlag.

## Patentansprüche

1. Mikrofluidische Mehrfach-Messkammeranordnung, die einen planaren Grundsensor (1) und eine strukturierte Polymerfolie (2) aufweist, wobei die dem Grundsensor (1) zugewandte Polymerfolienoberfläche unterschiedlich vertieft strukturierte Kammergeometrien besitzt, die parallel oder nacheinander angeordnet sind, **dadurch gekennzeichnet, dass** die Polymerfolie (2) innerhalb des mikrofluidischen Flussweges mindestens zwei parallel angeordnete Vertiefungen aufweist, die über dem Sensor angeordnet sind und in Kombination mit dem Grundsensor (1) Messkammern (3a, b, c) bilden, wobei die Kammerwandungen (4a, b, c) um und zwischen diesen Vertiefungen so konstruiert sind, dass sie auf der Oberfläche des Grundsensors (1) ohne stoffschlüssige Verbindung aufsitzen, wobei sie eine Höhe aufweisen, die mindestens einen passgenauen Kontakt mit der Oberfläche des Grundsensors (1) sichert und maximal fünf Prozent über der Höhe der Vertiefung der Messkammern liegt, wobei die strukturierte Polymerfolie (2) und der Grundsensor (1) außerhalb der vertieft strukturierten Kammergeometrien durch eine doppelseitige Klebefolie (5) oder durch Verschweißen oder Versiegeln miteinander formschlüssig verbunden sind.

2. Mikrofluidische Mehrfach-Messkammeranordnung nach Anspruch 1, **dadurch gekennzeichnet, dass** die Oberfläche des Grundsensors (1) eine Isolationsschicht (8) aufweist, die sich über die Fläche des Sensors (1) so erstreckt, dass lediglich die Messkammern ausgespart sind, so dass sich über den Kammerwandungen (4a, b, c) ein Isolationsschichtsteg (8a, b, c) befindet.

3. Mikrofluidische Mehrfach-Messkammeranordnung nach Anspruch 1 oder 2, **dadurch gekennzeichnet, dass** die Kammerwandungen (4a, b, c) eine Dicke zwischen 25 µm und 250 µm aufweisen.

4. Mikrofluidische Mehrfach-Messkammeranordnung nach einem der Ansprüche 1 bis 3, **dadurch gekennzeichnet, dass** die Kammerwandungen (4a, b, c) eine Höhe zwischen 5 µm und 150 µm aufweisen.

5. Mikrofluidische Mehrfach-Messkammeranordnung nach einem der Ansprüche 1 bis 4, **dadurch gekennzeichnet, dass** die zum Grundsensor aufliegenden Flächen der Kammerwandungen (4a, b, c) eben, halbkreisförmig oder spitz zulaufend sind.

6. Mikrofluidische Mehrfach-Messkammeranordnung nach einem der Ansprüche 1 bis 5, **dadurch gekennzeichnet, dass** die vertieft strukturierten Kammergeometrien Tiefen zwischen 5 µm und 150 µm aufweisen.

7. Mikrofluidische Mehrfach-Messkammeranordnung nach einem der Ansprüche 1 bis 6, **dadurch gekennzeichnet, dass** die Polymerfolie (2) und der Kunststoffsupport des Grundsensors (1) aus einem Polycarbonat, Polyester, Polypropylen, Polystyren oder einem Polyacrylat bestehen.

8. Mikrofluidische Mehrfach-Messkammeranordnung nach einem der Ansprüche 1 bis 7, **dadurch gekennzeichnet, dass** die vertiefende Strukturierung der Polymerfolie (2) mittels Heißprägeverfahren, eines photolithographischen Verfahrens, Laserablation, Mikrospritzguss oder Tiefziehverfahren erfolgte.

9. Mikrofluidische Mehrfach-Messkammeranordnung nach einem der Ansprüche 1 bis 8, **dadurch gekennzeichnet, dass** es sich bei dem Grundsensor (1) um einen elektrochemischen, mit gut leitfähigen inerten Strukturen beschichteten Kunststoffsupport handelt.

10. Mikrofluidische Mehrfach-Messkammeranordnung nach einem der Ansprüche 1 bis 8, **dadurch gekennzeichnet, dass** es sich bei dem Grundsensor um ein transparentes für optische Messungen geeignetes Kunststoffträgermaterial handelt.

## Claims

1. A microfluidic multiple measuring chamber arrangement, which comprises a planar base sensor (1) and a structured polymer film (2), wherein the polymer film surface facing the base sensor (1) has chamber geometries structured to differing depths, said chamber geometries being disposed in parallel or after one another, **characterised in that** the polymer film (2) comprises within the microfluidic flow path at least two recesses which are disposed in parallel and which are disposed over the sensor and form, in combination with the base sensor (1), measuring chambers (3a, b, c), wherein the chamber walls (4a, b, c) are constructed around and between these recesses in such a way that they sit on the surface of the base sensor (1) without a firmly bonded connection, wherein they have a height which ensures at least a precisely fitting contact with the surface of the base sensor (1) and lies at most five percent above the height of the recess of the measuring chambers, wherein the structured polymer film (2) and the base sensor (1) are connected to one another in a form-fit manner outside the depth-structured chamber geometries by means of a double-sided adhesive film (5) or by welding or sealing.

2. The microfluidic multiple measuring chamber arrangement according to claim 1, **characterised in that** the surface of the basis sensor (1) comprises an insulation layer (8), which extends over the area of the sensor (1), in such a way that only the measuring chambers are recessed, so that an insulation layer web (8a, b, c) is located over the chamber walls (4a, b, c).

3. The microfluidic multiple measuring chamber arrangement according to claim 1 or 2, **characterised in that** the chamber walls (4a, b, c) have a thickness between 25 µm and 250 µm.

4. The microfluidic multiple measuring chamber arrangement according to any one of claims 1 to 3, **characterised in that** the chamber walls (4a, b, c) have a height between 5 µm and 150 µm.

5. The microfluidic multiple measuring chamber arrangement according to any one of claims 1 to 4, **characterised in that** the areas of the chamber walls (4a, b, c) supported on the base sensor are flat, semicircular or pointed.

6. The microfluidic multiple measuring chamber arrangement according to any one of claims 1 to 5, **characterised in that** the depth-structured chamber geometries have depths between 5 µm and 150 µm.

7. The microfluidic multiple measuring chamber arrangement according to any one of claims 1 to 6, **characterised in that** the polymer film (2) and the plastic support of the base sensor (1) are made from a polycarbonate, polyester, polypropylene, polystyrene or a polyacrylate.

8. The microfluidic multiple measuring chamber arrangement according to any one of claims 1 to 7, **characterised in that** the deepening structuring of the polymer film (2) takes place by means of a hot-embossing process, a photolithographic process, laser ablation, micro-injection moulding or a deep-drawing process.

9. The microfluidic multiple measuring chamber arrangement according to any one of claims 1 to 8, **characterised in that** the base sensor (1) is an electrochemical plastic support coated with inert structures having good conductivity.

10. The microfluidic multiple measuring chamber arrangement according to any one of claims 1 to 8, **characterised in that** the base sensor is a transparent plastic support material suitable for optical measurements.

## Revendications

1. Dispositif à chambre de mesure multiple microfluidique qui présente un capteur de base (1) planaire et une feuille de polymère (2) structurée, la surface de la feuille de polymère tournée vers le capteur de base (1) possédant des géométries de chambre structurées à creux différents qui sont disposés parallèles ou l'un derrière l'autre, **caractérisé en ce que** la feuille de polymère (2) présente à l'intérieur de la voie d'écoulement microfluidique au moins deux cavités disposées en parallèle qui sont disposées au-dessus du capteur et forment des chambres de mesure (3a, b, c) en combinaison avec le capteur de base (1), les parois de chambre (4a, b, c) étant conçues autour et entre ces cavités de telle sorte qu'elles sont posées sur la surface du capteur de base (1) sans liaison par complémentarité de matière, présentant une hauteur qui assure au moins un contact exactement adapté avec la surface du capteur de base (1) et se situe au maximum cinq pour cent au-dessus de la hauteur de la cavité des chambres de mesure, la feuille de polymère (2) structurée et le capteur de base (1) étant reliés entre eux par complémentarité de forme à l'extérieur des géométries de chambre structurées en creux par une feuille adhésive double face (5) ou par soudure ou scellement.

2. Dispositif à chambre de mesure multiple microfluidique selon la revendication 1 **caractérisé en ce que** la surface du capteur de base (1) présente une couche d'isolation (8) qui s'étend sur la surface du capteur (1) de telle sorte que seules les chambres de mesure sont épargnées de sorte qu'une âme de couche d'isolation (8a, b, c) se trouve sur les parois de chambres (4a, b, c).

3. Dispositif à chambre de mesure multiple microfluidique selon la revendication 1 ou 2 **caractérisé en ce que** les parois de chambre (4a, b, c) présente une épaisseur entre 25 µm et 250 µm.

4. Dispositif à chambre de mesure multiple microfluidique selon une quelconque des revendications 1 à 3 **caractérisé en ce que** les parois de chambre (4a, b, c) présente une hauteur entre 5 µm et 150 µm.

5. Dispositif à chambre de mesure multiple microfluidique selon une quelconque des revendications 1 à 4 **caractérisé en ce que** les surfaces des parois de chambre (4a, b, c) en applique au capteur de base (1) sont planes, semi-circulaires ou effilées.

6. Dispositif à chambre de mesure multiple microfluidique selon une quelconque des revendications 1 à 5 **caractérisé en ce que** les géométries de chambre structurées en creux présentent des cavités entre 5 µm et 150 µm.

7. Dispositif à chambre de mesure multiple microfluidique selon une quelconque des revendications 1 à 6 **caractérisé en ce que** la feuille de polymère (1) et le support en matière plastique du capteur de base (1) sont composés de polycarbonate, de polyester, de polypropylène, de polystyrène ou d'un polyacrylate.

8. Dispositif à chambre de mesure multiple microfluidique selon une quelconque des revendications 1 à 7 **caractérisé en ce que** la structuration creusée de la feuille de polymère (2) a été réalisée au moyen d'un processus d'estampage à chaud, d'un processus photolithographique, d'une ablation au laser, de moulage par micro-injection ou un processus d'emboutissage.

9. Dispositif à chambre de mesure multiple microfluidique selon une quelconque des revendications 1 à 8 **caractérisé en ce que** concernant le capteur de base (1) il s'agit d'un support en matière plastique électrochimique revêtu de structures inertes bonnes conductrices.

10. Dispositif à chambre de mesure multiple microfluidique selon une quelconque des revendications 1 à 8 **caractérisé en ce que** concernant le capteur de base, il s'agit d'un matériau de support en matière plastique transparent adapté aux mesures optiques.
